# FASCICULE DE BREVET EUROPEEN

(11) **EP 1 248 648 B1**
(45) Date de publication et mention de la délivrance du brevet: **11.01.2012**
(21) Numéro de dépôt: 01905858.5
(22) Date de dépôt: 19.01.2001
(51) Int. Cl.: A61K 39/245, A61K 39/39, A61K 39/295, A61P 31/22, A61K 39/23, A61K 39/235, A61K 39/175

(54) **VACCINATION CONTRE L'HERPESVIROSE CANINE ET VACCINS**
IMPFUNG UND IMPFSTOFFE GEGEN HUNDEHERPESVIRUS
VACCINATION AGAINST CANINE HERPESVIRUS INFECTION AND VACCINES

(30) Priorité: 21.01.2000 FR 0000797
(43) Date de publication de la demande: 16.10.2002
(73) Titulaire: MERIAL, 69007 Lyon (FR)
(72) Inventeur: POULET, Hervé, F-69110 Sainte Foy les Lyon (FR)
(74) Mandataire: Nargolwalla, Cyra
(86) Numéro de dépôt international: PCT/FR2001/000189
(87) Numéro de publication internationale: WO 2001/052887

(56) Documents cités:
- WO-A-99/44633
- WO-A-99/51269
- US-A- 5 804 197
- ENGELS M ET COLL.: "Die Seroepizootologie des caninen Herpesvirusinfektion in der Schweiz und präliminäre Versuche mit einer Vakzine" ZENTRALBLATT FÜR VETERINÄRMEDIZIN REIHE B, vol. 27, no. 4, 1980, pages 257-267, XP000952687
- PIERSON P ET COLL.: "L'herpèsvirose en élevage canin : aspects cliniques et diagnostic" RECUEIL DE MEDECINE VETERINAIRE, vol. 174, no. 3-4, mars 1998 (1998-03), pages 87-94, XP000952697 cité dans la demande

## Description

La présente invention concerne la vaccination contre l'herpèsvirus canin. Elle a notamment trait à une méthode de vaccination, à des vaccins appropriés et à l'utilisation de vaccins dans le cadre de cette méthode de vaccination.

L'herpèsvirus canin (en anglais Canine Herpesvirus ou CHV) a été décrit pour la première fois en 1965 (L. E. Carmichael, Am. J. Vet. Res., 1965, 26, 803-814). L'infection par l'herpèsvirus canin a ensuite été identifiée dans de nombreux pays (U.S.A., France, Royaume-Uni, Suisse, Italie, Japon, Australie, Nouvelle-Zélande...). Le CHV est responsable de problèmes de reproduction dans les élevages canins et tout particulièrement de mortalité chez les chiots nouveau-nés qui peut occasionner des pertes importantes dans certaines collectivités. Il est également responsable d'avortements et de mortinatalité. L'infection par le CHV est par ailleurs suspectée de diminuer la fertilité des chiennes. La transmission du CHV se fait par voie vénérienne, par voie oronasale ou par voie trans-placentaire.

Le CHV appartient à la famille des *Herpesviridae* et à la sous-famille des *Alphaherpesvirinae.* A ce jour, un seul type de CHV a été identifié. Il s'agit d'un virus enveloppé contenant une molécule d'ADN double brin. L'enveloppe présente plusieurs glycoprotéines d'origine virale. Les glycoprotéines gB, gC et gD sont responsables de l'induction d'anticorps neutralisants chez la souris.

Le CHV est antigéniquement proche de l'herpèsvirus félin (J.A. Limcumpao et al., Arch. Virol., 1990, 111, 165-176) mais sans neutralisation croisée significative (X. Xuan et al., Arch. Virol., 1992, 122, 359-365).

Le CHV est fortement dépendant de son hôte et ne se multiplie que sur des cellules d'origine canine (Pierson et al., recueil de Médecine Vétérinaire, mars/avril 1998, 174 n°3/4. 87-94).

Les chiots nouveau-nés sont très sensibles à l'infection par le CHV lors des 3 premières semaines de vie. La contamination intervient lors de la naissance ou dans les premiers jours de vie. La vaccination des chiots n'est en conséquence pas possible. Les seuls moyens de protection disponibles sont les mesures sanitaires comme le réchauffement des chiots avec une lampe à infrarouges et la sérothérapie. Le CHV est cependant peu immunogène et il est difficile de préparer un bon sérum (L. E. Carmichael, J. Am. Vet. Med. Assoc., 1970, 156, 1714-1721).

Dans les conditions naturelles, le chiot peut être protégé par les anticorps neutralisant le CHV présents dans le colostrum des chiennes séropositives : en l'absence de ces anticorps, le chiot est très sensible à l'infection par le CHV (D. L. Huxsoll et I. E. Hemelt, J. Am. Vet. Med. Assoc., 1970, 156, 1706-1713). Les animaux à risque sont les chiots nés de chiennes non infectées en contact d'animaux excréteurs, et les chiots nés de chiennes infectées latentes mais séronégatives ou faiblement séropositives. Les anticorps neutralisants le CHV persistent peu de temps, et un animal peut être infecté et séronégatif.

Le CHV provoque chez les chiots de moins de 3 semaines d'âge une herpèsvirose néonatale souvent fatale. L'incubation est rapide et les chiots meurent en quelques jours le plus souvent sans autres symptômes qu'une hypothermie brutale et des troubles nerveux foudroyants, dans la grande majorité des cas avant l'âge de 5 jours. Dans les formes moins aiguës, l'hypothermie est suivie de symptômes tels qu'anorexie, dépression, bradycardie, hypoglycémie, oedème sous-cutané, érythème et papules ventrales, selles molles gris-jaunâtre, douleurs abdominales, vomissements, plaintes incessantes, pédalage et mort en 24 à 72 heures en opisthotonos. L'examen nécropsique peut révéler une splénomégalie, un épanchement séreux dans les cavités pleurales et péritonéale et des pétéchies viscérales (Pierson et al., recueil de Médecine Vétérinaire, mars/avril 1998, 174 n°3/4, 87-94). Cet article rappelle qu'aucun vaccin contre l'herpèsvirose canine n'est actuellement commercialisé et suggère qu'il serait utile de pouvoir vacciner les reproducteurs infectés latents afin d'atténuer la ré-excrétion du virus sauvage.

Delisle F., dans son article (Rec. Med. Vet., 1982, 158, 669-676), propose en revanche de vacciner les chiennes avant la gestation avec un vaccin inactivé ou d'injecter un sérum aux chiots dès la naissance. Elle rappelle toutefois qu'aucun vaccin commercial n'est disponible. Voir aussi L. E. Carmichael, J. Am. Vet. Med. Assoc., 1970, 156, 1714-1721. De son coté Appel A., dans son article (« Canine Herpesvirus » dans Virus Infections of Vertebrates, vol.1, MC Horzinek, Ed. MJ Appel Elsevier Science Publisher, 1987, 5-15), propose de vacciner des chiennes avant ou au tout début de la gestation avec un vaccin inactivé.

H. Poulet et P. Dubourget (Point Vét, 1993, 25, 69-75) ont quant à eux suggéré en 1992 en termes généraux la vaccination de la mère avec un rappel en fin de gestation. Les auteurs rappellent aussi que des essais à partir de variants atténués ont été effectués (L. E. Carrnichaet et al., Infection and Immunity, 1978, 20(1), 108-114 ; US-A-4,213,965 : vaccin vivant atténué, composé d'un variant thermosensible du virus CHV, dit « de petites plages »), mais que ces essais n'ont pas eu de résultats probants et n'ont pas donné lieu à la commercialisation de vaccins.

Anvik J. O., dans son article (Vet. Med., April 1991, 4, 394-403), précise que la production d'anticorps neutralisants chez la mère et en conséquence la protection de la portée est imprédictible en raison de la faible immunogénicité du virus CHV.

Devant la difficulté de vaccination, Pierson et al. (recueil de Médecine Vétérinaire, mars/avril 1998, 174 n°3/4, 87-94) sont même allés jusqu'à proposer de vacciner la mère avec un vaccin inactivée contre le coryza du chat (FHV) ou d'administrer des anticorps anti-FHV aux chiots dès la naissance.

A ce jour aucune des hypothèses de **vaccination** n'a été confirmée, aucun vaccin n'est disponible dans le commerce et aucune publication ne fait état de résultats de vaccination convaincants annonciateurs de la commercialisation prochaine d'un tel vaccin.

En outre chez les femelles gestantes, l'infection au CHV peut conduire à une résorption embryonnaire, une momification foetale, un avortement ou une mise bas prématurée, une mortinatalité, une mortalité néonatale. Des lésions placentaires peuvent apparaître.

Le document WO 99 44633 divulgue un vaccin recombinant contre CHV comprenant un vecteur viral incorporant et exprimant un vive au moins l'un des gènes de glycoprotéine d'enveloppe, à savoir gB, gC et gD de CHV. Ce vaccin recombinant peut être administré avec au moins un adjuvant choisi parmi les polymères acryliques ou méthacryliques et les copolymères d'anhydride maléique et de dérivé alcényle. Toutefois, ce document ne divulgue aucune information sur les modalités de vaccination contre l'herpèsvirose canin qui permettraient d'induire une immunité et une protection des chiennes en gestation et des chiots à leur naissance.

Le document US-A-5 804 197 décrit un vecteur recombinant CHV exprimant des gènes hétérologues, des génomes recombinants de CHV et leurs utilisations. En particulier, ce document recommande de déléter les gènes codant pour les glycoprotéines gB, gD et gC.

Bien que les auteurs suggèrent l'inoculation de chiennes gestantes afin d'induire une protection passive chez les chiots, ils restent silencieux sur les modalités d'inoculation. Par ailleurs, il semble important de préciser que l'efficacité d'un tel virus rebombinant en tant que préparation thérapeutique capable d'induire une protection chez le chien n'a pas été démontrée.

L'article scientifique d'Engels et al (Zbl. Vet. Med. 1980, 8,27:257-267) décrit l'utilisation d'un vaccin contre CHV concernant des particules virales inactivées aux rayons gamma chez des chiens adultes. Les auteurs recommandent de vacciner des chiennes reproductrices deux à trois fois avant la saillie. Les auteurs suggèrent également que les chiennes gestantes devraient recevoir une dernière inoculation trois à quatre semaines avant la date de mise-bas.

Alors que des vaccins contre différents herpèsvirus existent chez d'autres espèces animales dont le chat, la vaccination efficace contre l'herpèsvirose canine n'a jamais pu être proposée.

La présente invention a pour objectif la mise au point d'une méthode de vaccination contre l'herpèsvirose canine permettant de protéger les chiots.

Un autre objectif de l'invention est l'élaboration de vaccins, efficaces et pharmaceutiquement acceptables, utilisables dans le cadre de cette méthode de vaccination.

La déposante a trouvé de façon surprenante qu'il est possible de vacciner les chiennes gestantes et les nouveaux-nés en administrant un vaccin pendant la période de gestation et en particulier le plus près possible de la mise bas de façon à avoir un taux d'anticorps anti-CHV élevé chez la femelle gestante au moment de la mise bas, ce taux assurant la protection du chiot à la naissance par transmission des anticorps maternels lors de l'allaitement. En outre ces anticorps se maintiennent substantiellement chez le chiot pendant les premières semaines de sa vie, période où il est sensible à la maladie (3 à 4 semaines). Un titre en anticorps neutralisant chez la chienne égal ou supérieur à 0,9 log10 est préféré et plus particulièrement un titre égal ou supérieur à 1,2 log10. Les titres indiqués sont calculés d'après la méthode décrite dans l'exemple 7. La vaccination chez la chienne amène d'autre part une réduction de la pression d'infection chez le chiot. La déposante a observé par ailleurs chez les chiennes vaccinées en milieu contaminé un poids des chiots supérieur à la naissance et une tendance à un taux de gestation plus élevé. Ces phénomènes peuvent s'expliquer par une réduction ou une absence de lésions placentaires chez les chiennes vaccinées (A. Hashimoto et al., Am. J. Vet. Res., 1979, 40(9), 1236-1240 ; A. Hashimoto et al., Am. J. Vet. Res., 1982, 43(5), 844-850).

Les objectifs de l'invention déjà mentionnés, ainsi que d'autres, sont obtenus à l'aide d'une méthode de vaccination de la chienne gestante comprenant l'administration d'un vaccin chez une femelle gestante le plus près possible de la mise bas, notamment pendant le dernier tiers de gestation (la durée moyenne de gestation chez la chienne est d'environ 63 jours) environ 10 jours avant la date présumée de mise bas, de façon à avoir un taux d'anticorps anti-CHV élevé chez la femelle gestante à la mise bas, induisant une protection des chiots par transfert des anticorps maternels lors de l'allaitement. Ces anticorps se maintiennent substantiellement pendant les premières semaines de vie du chiot, période où il est sensible à la maladie (3 à 4 semaines).

Par définition, un antigène, en quantité efficace dans le vaccin selon l'invention, permet d'induire un titre élevé et protecteur d'anticorps dans les conditions du protocole chez la femelle gestante et le nouveau-né. Il peut s'agir d'antigène comprenant du virus CHV inactivé, <du virus CHV une ou plusieurs sous-unités du virus CHV .

De préférence, le vaccin est destiné à produire chez la femelle gestante, au moment de la mise bas, un taux d'anticorps anti-CHV (déterminé conformément à l'exemple 7) supérieur ou égal à environ 0,9 log10, de préférence supérieur ou égal à environ 1,2 log10.

Notamment dans le cas d'un animal n'ayant jamais été vacciné contre le CHV, on procède à une (ou plusieurs) autre administration de vaccin anti-CHV avant administration décrite ci-dessus afin d'induire la réponse immunitaire chez l'animal. L'intervalle entre ces deux administrations doit être suffisant pour induire un effet rappel, ce qui en général demande au moins de 2 à 3 semaines. La primo-administration du vaccin se fait environ 10 jours après la saillie. Suivant une modalité préférée, on reproduit ce schéma de vaccination pour chaque mise bas.

L'administration, ou l'une au moins des administrations supplémentaires peuvent aussi être réalisés avec un vaccin ayant une composition différente de celle intervenant au plus près de la mise bas, la différence pouvant toucher l'antigène et/ou la formulation du vaccin.

L'administration du vaccin peut se faire notamment par la voie parentérale, de préférence par la voie sous-cutanée ou intramusculaire.

Les volumes de dose peuvent être notamment compris entre 0,2 et 2 ml, de préférence de l'ordre de 1 ml.

L'invention a aussi pour objet l'utilisation d'antigène CHV pour la préparation d'un vaccin contre l'herpèsvirose canine destiné à être administré selon le protocole décrit ci-dessus.

L'homme de l'art possède les compétences nécessaires pour définir précisément les doses de chaque vaccin en fonction du type de vaccin.

Lorsque plusieurs administrations sont pratiquées chez l'animal, les vaccins utilisés peuvent être de différents types, choisis notamment parmi ceux précédemment cités.

L'invention a aussi pour objet des vaccins inactivés ou des vaccins de sous-unités, utilisables notamment dans le cadre de cette méthode de vaccination.

L'utilisation de sous-unités CHV n'a été, décrite que chez la souris (J. A. Limcumpao et al., J. Vet. Med. Sci., 1991, 53(3), 423-432). Les données obtenues chez la souris ne peuvent cependant pas être extrapolées à l'espèce canine comme le précise ce document. Bien que les titres en anticorps obtenus avec les glycoprotéines gp145/112, gp80 et gp47 purifiées induisent une réponse en anticorps détectable, il n'est pas toutefois possible de déterminer si ce titre en anticorps peut se traduire par une protection car la souris n'étant pas sensible au virus CHV il n'est pas possible de développer un modèle d'épreuve virulente chez cette espèce. Egalement aucune donnée n'est disponible concernant l'immunité cellulaire induite par CHV. Par ailleurs la relation entre le titre en anticorps obtenu chez la souris et celui obtenu chez le chien n'est pas connue et ces titres peuvent se révéler différents. Ainsi il a été publié que des lapins immunisés avec la glycoprotéine gl d'herpès bovin de type 1 ont un titre neutralisant plus élevé que les lapins immunisés avec gIII, alors que chez le bovin la glycoprotéine gIV induit le titre neutralisant le plus élevé et que la glycoprotéine gl est la moins immunogène. Ces données montrent la difficulté de transposition des résultats des animaux de laboratoire à l'espèce destinataire.

Conformément à l'invention, l'utilisation d'un vaccin inactivé ou de sous-unités permet la vaccination de la chienne pendant la phase de gestation sans risque pour la chienne et sa progéniture. La vaccination avec rappel en fin de gestation est le moyen d'assurer un titre optimal en anticorps neutralisants à la naissance. Ce titre conditionne la protection obtenue par transfert des anticorps neutralisants de la chienne à ses chiots en début d'allaitement.

La culture et la propagation du virus CHV se fait de préférence sur des cellules canines primaires ou de lignée, plus particulièrement sur cellules rénales canines de lignée de type MDCK (en anglais Madin-Darby Canine Kidney ou MDCK accessible auprès de l'American Type Culture Collection (ATCC) sous le numéro CCL-34) notamment avec une multiplicité d'infection (moi) de 0,1 à 0,001 doses infectantes 50 % en culture de cellules (DICC₅₀) par cellule, de préférence 0,01 DICC₅₀/cellule. La récolte du virus CHV a lieu 3 à 4 jours plus tard. On peut obtenir un titre viral d'environ 10⁶ à 10⁸ DICC₅₀/ml et en général de 10^{6,5} à 10^{7,5} DICC₅₀/ml.

Pour obtenir un vaccin inactivé, le virus CHV est inactivé, notamment après récolte et clarification, par un traitement chimique (e.g. formol ou formaldéhyde, β-propiolactone, éthylèneimine, éthylèneimine binaire (BEI)) et/ou thermique. De préférence, le virus selon l'invention est inactivé par action de l'éthylèneimine. Les particules virales peuvent être concentrées par des techniques classiques de concentration, notamment par ultrafiltration et/ou purifiées par des moyens de purification classiques, notamment les techniques de gel-filtration, d'ultracentrifugation sur gradient de saccharose ou de précipitations sélectives en particulier en présence de polyéthylène glycol (PEG).

Le vaccin de sous-unités est de préférence un vaccin sous-unités d'extraction, c'est-à-dire qu'il est préparé à partir du virus entier ou d'une fraction importante de ce virus (par exemple après élimination de la capside). En particulier, ce vaccin peut comprendre les différentes glycoprotéines d'enveloppe ou un mélange de glycoprotéines d'enveloppe. La préparation du vaccin de sous-unités est de préférence réalisée à partir d'une suspension virale inactivée, notamment inactivée comme décrit ci-dessus. De manière préférée, pour obtenir un vaccin de sous-unités, contenant principalement les glycoprotéines d'enveloppe, la suspension de particules virales, de préférence inactivées obtenue précédemment, est soumise à l'action d'un détergent approprié, notamment non ionique, de préférence un alcool éthoxylé ayant avantageusement un HLB compris entre 12 et 15. Les capsides virales sont ensuite éliminées notamment par ultracentrifugation. D'autres méthodes équivalentes, permettant d'éliminer les capsides et de recueillir les glycoprotéines d'enveloppe sont à la disposition de l'homme du métier.

Pour l'élaboration d'un vaccin inactivé ou d'un vaccin de sous-unité, l'antigène est repris dans un véhicule ou excipient acceptable au plan vétérinaire et additionné d'un adjuvant acceptable au plan vétérinaire. La quantité d'antigène équivaut notamment à un équivalent titre avant inactivation d'environ 10⁵ à environ 10^{9.5} DICC₅₀ par dose, notamment d'environ 10⁵ à environ 10⁹ par dose. Plus particulièrement, pour un vaccin inactivé, le titre avant inactivation est compris entre environ 10⁵ est environ 10⁹ DICC₅₀ par dose, notamment entre environ 10⁶ et environ 10⁸ par dose. Plus particulièrement, pour un vaccin sous-unités, le titre avant inactivation est compris entre environ 10⁶ et environ 10^{9.5}, en particulier entre environ 10⁷ et environ 10⁹, de préférence entre environ 10^{7.5} et environ 10^{8.5}, par dose. De préférence, lorsqu'il s'agit d'un vaccin sous-unités, il présente un titre en glycoprotéine gB, mesuré en ELISA, d'environ 0,01 µg à environ 30 µg, en particulier d'environ 0,1 µg à environ 10 µg, et de préférence d'environ 0,3 µg à environ 3 µg, par dose.

Les vaccins selon l'invention sont conservés et stockés soit sous forme formulée à 5°C, soit sous forme lyophilisée, de préférence avec un stabilisateur, notamment du SPGA (saccharose, phosphate, glutamate, albumine, EP-B1-0,008,255). Ces vaccins peuvent être repris ultérieurement en solvant (véhicule ou excipient et/ou adjuvant).

Pour adjuver les vaccins selon l'invention, on l'hydroxyde d'alumine, (2) un polymère de l'acide acrylique formule la préparation immunogène ou vaccin sous forme d'une émulsion huile-dans-l'eau en particulier l'émulsion SPT décrite à la page 147 de « Vaccine Design, The Subunit and Adjuvant Approach » edited by M. Powell, M. Newman, Plenum Press 1995, et "émulsion MF59 décrite à la page 183 du même ouvrage. (Squelane, Pluronic L101^{®}, Tween 80^{®})

L'émulsion huile-dans-l'eau est à base d'huile de paraffine liquide légère (type Pharmacopée européenne) et peut comprendre des esters d'acides ou d'alcools à groupement alkyle linéaire, plus particulièrement les huiles végétales, l'oléate d'éthyle, le di(caprylate / caprate) de propylène glycol, le tri(caprylate / caprate) de glycérol, le dioléate de propylène glycol ; des esters d'acides ou d'alcools gras ramifiés, en particulier des esters de l'acide isostéarique. L'huile est utilisée en association avec des émulsifiants pour former l'émulsion. Les émulsifiants sont de préférence des tensio-actifs non ioniques en particulier les esters de sorbitan, de mannide (e.g. oléate d'anhydromannitol), de glycérol, de polyglycérol, de propylène glycol et d'acide oléique, isostéarique, ricinoléique, hydroxystéarique, éventuellement éthoxylés, les blocs copolymères polyoxypropyléne-polyoxyéthylène en particulier les Pluronic®, notamment L121.

Les polymères de l'acide acrylique ou méthacrylique sont réticulés notamment par des éthers polyalcényliques de sucres ou de polyalcools. Ces composés sont connus sous le terme carbomère (Pharmeuropa vol. 8, n° 2, juin 1996). L'homme de l'art peut aussi se référer à US-A-2,909,462 (incorporé par référence) décrivant de tels polymères acryliques réticulés par un composé polyhydroxylé ayant au moins 3 groupes hydroxyle, de préférence pas plus de 8, les atomes d'hydrogène d'au moins trois hydroxyles étant remplacés par des radicaux aliphatiques insaturés ayant au moins 2 atomes de carbone. Les radicaux préférés sont ceux contenant de 2 à 4 atomes de carbone, e.g. vinyles, allyles et autres groupes éthyléniquement insaturés. Les radicaux insaturés peuvent eux-mêmes contenir d'autres substituants, tel que méthyl. Les produits vendus sous la dénomination Carbopol® (BF Goodrich, Ohio, USA) sont particulièrement appropriés. Ils sont réticulés par un allyl saccharose ou par de l'altylpentaérythritol. Parmi eux, on peut citer les Carbopol® 974P, 934P et 971P.

Parmi les copolymères d'anhydride maléique et de dérivé alcényle, on préfère les EMA® (Monsanto) qui sont des copolymères d'anhydride maléique et d'éthylène, linéaires ou réticulés, par exemple réticulés par du divinyléther. On peut se référer à J. Fields et al., Nature, 188, 778-780, 4 juin 1960 . Sur le plan de leur structure, les polymères d'acide acrylique ou méthacrylique et les EMA® sont formés de préférence de motifs de base de formule suivante : 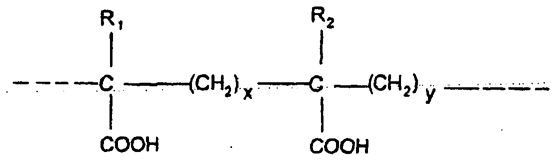 dans laquelle :
- R₁ et R₂. identiques ou différents, représentent H ou CH₃
- x = 0 ou 1, de préférence x = 1
- y = 1 ou 2, avec x + y = 2

Pour les EMA®, x = 0 et y = 2. Pour les carbomères, x = y = 1.

La concentration en polymère dans la composition vaccinale finale sera de 0,01 % à 1,5 % PN, plus particulièrement de 0,05 à 1 % PN, de préférence de 0,1 à 0,4 % PN.

Une forme de vaccin particulièrement préférée comprend un vaccin inactivé ou de sous-unités formulé sous forme d'une émulsion huile dans l'eau comprenant de l'oléate d'anhydromannitol, de l'acide oléique éthoxylé et de l'huile de paraffine liquide légère. En particulier, une méthode d'immunisation des chiennes gestantes selon l'invention comprend l'administration d'un tel vaccin. Deux injections de préférence sous-cutanées sont réalisées, en particulier selon le schéma décrit plus haut, avec la première injection pendant le premier tiers de la gestation de préférence environ 10 jours après la date de la saillie et la deuxième injection pendant le dernier tiers de préférence environ 10 jours avant la date présumée de la mise bas.

L'invention a aussi pour objet le procédé de préparation du vaccin inactivé ou de sous-unités décrit.

L'invention a aussi pour objet des vaccins multivalents comprenant la valence herpèsvirus canin inactivée ou de sous-unités, et au moins une valence pour au moins un autre pathogène canin, dans un véhicule ou excipient acceptable au plan vétérinaire et avec un adjuvant, comprenant une huile de paraffine légére liquide

Lesdits pathogènes sont notamment choisis parmi le groupe comprenant le virus de la maladie de Carré, le parvovirus canin et l'adénovirus canin, mais d'autres valences peuvent aussi être associées.

Les vaccins CHV inactivés ou de sous-unités selon l'invention peuvent être administrés simultanément dans la même préparation ou successivement dans des préparations différentes avec la ou les autres valences canines qui peuvent être sous forme de vaccins vivants atténués, inactivés, sous-unitaires, recombinés ou polynucléotidiques.

L'invention a donc également pour objet un kit ou ensemble de vaccin multivalent comprenant, conditionnées séparément, une valence CHV selon l'invention dans un véhicule ou excipient acceptable au plan vétérinaire, avec un adjuvant, comprenant une huile de parafine liquide légére et au moins une valence d'au moins un autre pathogène canin. La valence CHV selon l'invention peut servir de solvant pour l'autre valence canine, notamment pour une valence atténuée, recombinée ou polynucléotidique se présentant sous forme lyophilisée.

L'invention va être maintenant décrite plus en détail à l'aide de modes de réalisation pris à titre d'exemples non limitatifs.

### Exemples :

### Exemple 1 : Souche virale

La souche d'herpèsvirus canin utilisée est la souche F205 (L. E. Carmichael, Am. J. Vet. Res., 1965, 26, 803-814), disponible auprès de l'American Type Culture Collection (ATCC) sous le numéro VR-647.

### Exemple 2 : Amplification de l'herpèsvirus canin

Des cellules de lignée canine (Madin-Darby Canine Kidney ou MDCK disponible auprès de l'ATCC sous le numéro CCL-34) sont mises en culture en flacons roulants de 2 litres (850 cm²) avec du milieu Dulbecco modified Eagle's minimum essential medium (DMEM - Gibco BRL) supplémenté de 5 % de sérum de veau foetal (Bayer Diagnostic) et de gentamicine à 50 mg/l à raison de 200 000 cellules par ml sous un volume de 350 ml. Les cellules sont cultivées à +37°C en atmosphère contenant 5% de CO₂.

Après 3 ou 4 jours la couche cellulaire arrive à confluence. Le milieu de culture est alors remplacé par du milieu DMEM sans sérum mais toujours additionné de 50 mg/l de gentamicine et les cellules sont inoculées avec l'herpèsvirus canin (exemple 1) avec une multiplicité d'infection (moi) de 0,01 DICC₅₀/cellule. La culture virale est maintenue à 37°C.

Lorsque l'effet cytopathique (ECP) est complet (environ 96 heures après le début de la mise en culture), la suspension virale est récoltée et congelée à -70°C. Plusieurs passages d'amplification cellulaire peuvent être réalisés préalablement à l'inoculation virale en fonction de la quantité de virus souhaitée. Les passages cellulaires et la culture virale peuvent également être réalisés en cultivant les cellules sur microporteurs en biogénérateur.

Le virus est conservé jusqu'aux étapes suivantes à 5°C.

Le titre du virus CHV à la récolte est 10^{7.2} DICC₅₀ par ml.

### Exemple 3 : Titrage du virus CHV

Le titrage du virus CHV est réalisé en microplaques de 96 cupules en milieu F15 additionné de 5% de sérum de veau foetal et de gentamicine 50 mg/l. Les dilutions avec une raison d'ordre 3 de la suspension virale sont mélangées à une suspension de cellules MDCK dans la microplaque à raison de 0,10 ml de dilution de virus pour 100 000 cellules sous 0,15 ml par cupule. Après de 4 à 5 jours d'incubation à 37°C avec 5% de CO₂, les cupules présentant un ECP généralisé sont comptabilisées et le titre est calculé en doses infectieuses pour culture de cellules 50 % (DICC₅₀) par la méthode de Kârber.

### Exemple 4 : Inactivation du virus

Le virus CHV amplifié comme cela est décrit dans l'exemple 2 est inactivé par l'éthylèneimine à la concentration d'environ 12 mM à 37°C pendant 6 heures.

L'éthylèneimine est préparée extemporanément en dissolvant 28,0 g de soude en pastille dans 200 ml d'eau distillée et en ajoutant 68,1 g de bromoéthylamine (BEA) correspondant à une solution d'éthylèneimine 1,2 M (H. Bahnemann, Arch. Virol., 1975, 47, 47-56). La suspension virale inactivée est concentrée 100 fois sur cassette d'ultrafiltration type Ultrasette avec un seuil de coupure de 300 kDa (Filtron). Le virus inactivé concentré peut être congelé et stocké à -40°C.

### Exemple 5 : Extraction des glycoprotéines virales

Le virus inactivé (exemple 4) est clarifié par centrifugation à 2500 g pendant 30 min. Le liquide surnageant est ensuite concentré 50 fois sur cassette d'ultrafiltration type Ultrasette avec un seuil de coupure de 300 kDa (Filtron). Le concentrat est additionné de polyéthylène glycol 8000 (PEG 8000) à 8% PN et de chlorure de sodium 4% PN. Après un contact de 16 heures à 4°C, le virus précipité est sédimenté par centrifugation à 2500 g pendant 60 min, puis repris en tampon phosphate (PBS : NaCl 8 g/l ; KCl 0,2 g/l ; KH₂PO₄ 0,2 g/l ; Na₂HPO₄, 2 H₂O 1,44 g/l). Puis repris en tampon phosphate isotonique à raison d'un centième du volume initial. Le concentré viral subit ensuite une ultracentrifugation zonale sur coussin de saccharose (35 % et 60 % P/P en PBS, pendant 60 min, à 200 000 g) avec un rotor zonal ou un rotor à angle fixe Ti45. Le virus est récolté à l'interface et est dilué environ au quart en PBS. Puis ajouter un alcool cétylique 10 OE (alcool éthoxylé d'HLB 13) à la concentration de 1,5% PN, incuber une heure à 37°C. Les capsides sont ensuite sédimentées par ultracentrifugation pendant une heure à 230 000 g avec un rotor Ti45. Le surnageant contenant les glycoprotéines est recueilli. La solution de glycoprotéines peut éventuellement être concentrée par ultrafiltration.

La solution de glycoprotéines purifiées est conservées à -40°C jusqu'à formulation du vaccin.

### Exemple 6 : Formulation des vaccins

L'antigène inactivé concentré (exemple 4) ou la solution de sous-unités (exemple 5), après décongélation, est dilué(e) en tampon PBS en fonction de la quantité requise d'antigènes par dose.

Le vaccin adjuvé est préparé de la manière suivante : 167 ml de phase aqueuse constituée de 8 ml d'antigène viral inactivé ou sous-unité et de 159 ml de PBS sont émulsionnés dans 83 ml de phase huileuse contenant 7% p/v d'oléate d'anhydromannitol, 8% p/v d'acide oléique éthoxylé à 11 OE (oxyde d'éthylène) et 85% v/v d'huile de paraffine liquide légère (type Pharmacopée européenne) à l'aide d'un émulseur à turbine Silverson à 32°C pendant 2 minutes. Le vaccin formulé sous forme d'émulsion huile-dans-eau est ensuite stocké à 5°C.

Une méthode alternative pour préparer le vaccin consiste à mettre en émulsion par trois passages à travers un homogénéiseur haute pression modèle Y110 (Microfluidics Corp.) à une pression de 600 bars et une température comprise entre 30 et 40°C le mélange 5% p/v squalane, 2,5% p/v Pluronic® L121, 0,2% p/v d'un ester d'acide oléique et d'un anhydrosorbitol éthoxylé à 20 OE, 92,3% v/v de l'antigène viral dilué au 1/30^{ème} en tampon PBS après décongélation. Le vaccin formulé sous forme d'émulsion huile-dans-eau est ensuite stocké à 5°C.

Une autre méthode alternative consiste à réaliser une solution à 0.4% p/v de Carbopol® 974P en eau physiologique (NaCl 9 g/l). Le pH est ajusté à 7,3-7,4 avec de la soude. Cette solution de Carbopol est ensuite mélangée à partie égale à l'antigène viral dilué au 1/16^{ème} après décongélation. Le vaccin formulé sous d'une suspension est ensuite conservé à 5°C.

0,5 ml d'une solution de sous-unités (exemple 5), après décongélation, diluée en tampon PBS au 1/15^{ème} est ajoutée à 0,5 ml de substrat de lyophilisation SPGA (saccharose, phosphate, glutamate, albumine, EP-B1-0,008,255). Ce mélange est réparti en flacon et lyophilisé puis conservé à 5°C. Le diluant est constitué par les adjuvants précédemment décrit en incorporant uniquement du tampon PBS dans la phase aqueuse.

### Exemple 7: Titrage des anticorps neutralisants anti-CHV.

Les sérums à titrer sont testés pour leur aptitude à neutraliser le virus CHV. Les sérums prélevés sur les animaux sont dilués en cascade au tiers avec du milieu DMEM additionné de 5% de sérum de veau foetal dans des plaques à 96 puits pour culture cellulaire. A 0,05 ml du sérum dilué sont ajoutés 0,05 ml de milieu de culture contenant approximativement 200 DICC₅₀/ml de CHV. Ce mélange est incubé pendant 2 heures à 37°C en étuve en atmosphère contenant 5% CO₂.
0,15 ml d'une suspension de cellules MDCK contenant environ 100 000 cellules par ml est ensuite ajoutée à chaque mélange. L'effet cytopathique (ECP) est observé par microscopie à contraste de phase après 5 jours de culture à 37°C en atmosphère contenant 5% CO₂. Les titres neutralisants de chaque sérum sont calculés d'après la méthode de Kärber. Les titres sont donnés sous la forme de la dilution la plus importante inhibant l'effet cytopathique pour 50 % des cupules.
Les titres sont exprimés en log10 VN50 (Neutralisation de 50% du Virus). Chaque sérum est titré au moins deux fois, de préférence quatre fois.

### Exemple 8 : Efficacité

12 chiennes Beagle EOPS (Exmpt d'Organisme Pathogéne Specifique) non vaccinées ont été incluses dans l'étude et réparties par randomisation en 2 groupes de 6 animaux. Les chiennes du premier groupe sont vaccinées avec le vaccin CHV de sous-unités adjuvé avec l'émulsion huile-dans-eau à base d'huile de paraffine (exemple 6), les chiennes de l'autre groupe avec un vaccin placebo.

Les chiennes ont été vaccinées à 2 reprises par voie sous-cutanée environ 10 jours après saillie, puis environ 10 jours avant la date présumée de la mise-bas. Le lyophilisat repris par 1 ml de solvant huileux est administré aux chiennes. Ceci correspond à un équivalent titre de 10^{7.7} DlCC₅₀ avant inactivation.

Après la mise-bas, les chiots nouveau-nés sont éprouvés à l'âge de 3 jours avec 50 à 100 DICC₅₀ d'une souche virulente de CHV, par exemple F205 isolée par L. E. Carmichael (Am. J. Vet. Res., 1965, 26, 803-814) ou CHV 270 (H. Hill, Am. J. Vet. Res.,1974, 35, 669-673) ou CHV C4012SE, par voie oronasale (0,5 ml par voie orale et 0,25 ml dans chaque narine). Les chiots sont ensuite observés pendant 3 semaines. Les signes cliniques et la mortalité sont relevés. Après leur décès, tous les chiots sont autopsiés. Les lésions macroscopiques caractéristiques de l'herpèsvirose canine sont recherchées au niveau des reins, rate; foie, poumons, tractus digestif, coeur et ganglions mésentériques, et des prélèvements sont réalisés pour un isolement de CHV sur cellules MDCK. Le diagnostic d'herpesvirose canine est basé sur la mort du chiot, la présence de lésions macroscopiques caractéristiques et confirmé par un isolement viral.

Toutes les chiennes vaccinées ont séroconverti et ont un titre élevé en anticorps neutralisants au moment de la mise bas. Ces titres sont mesurés par la méthode décrite dans l'exemple 6 et sont exprimés en log10 VN50.

| Titres SN moyens anti-CHV chez les chiennes | Jour de la primo-vaccination | Jour du rappel | Jour de l'épreuve | 3 semaines après épreuve |
|---|---|---|---|---|
| groupe contrôle | < 0,24 +/- 0,00 | < 0,24 +/- 0,00 | < 0,29 +/- 0,13 | < 1,36 +/- 0,65 |
| groupe vacciné | < 0,35 +/- 0,17 | < 1,04 +/- 0,34 | 1,76 +/- 0,20 | 2.11 +/- 0,51 |

Parmi les chiots nés des mères vaccinées, aucun n'est mort d'herpesvirose canine (0/27). Aucun virus CHV n'a été ré-isolé de ces chiots.

Parmi les chiots nés de mère non vaccinées (groupe placebo), un taux de mortalité due au CHV de 62% (18/29) a été observé. De l'isolement viral est également observé chez 55% de ces chiots (16/29) : les prélèvements provenant de 2 chiots ont été contaminés par des bactéries et n'ont pu être testés pour l'isolement viral de CHV.

La différence du taux de mortalité entre les 2 groupes est hautement significative (test exact de Fisher ; p<0.0001).

Des chiennes Yorkshire réparties par randomisation en 2 groupes sont maintenues en milieu infecté. Les 14 chiennes du premier groupe sont vaccinées avec le vaccin CHV de sous-unités adjuvé avec une émulsion huile-dans-eau à base d'huile de paraffine (exemple 6), les 6 chiennes de l'autre groupe avec un vaccin placebo. Le protocole de vaccination est identique à ce qui est décrit plus haut.

A leur naissance, le poids des chiots est mesuré.

Le poids des 28 chiots nés d'une mère vaccinée est sensiblement plus élevé (poids moyen de 141,8 g) que celui des 14 chiots nés d'une mère non vacciné (poids moyen de 85,0 g), soit un gain pondéral de 166,8%. La différence est significative (Kruskal-Wallis, p<0,01).

Les résultats montrent en milieu contaminé une tendance à augmenter le taux de gestation chez les chiennes vaccinées.

Le taux de gestation est de 82% chez les chiennes vaccinées (50/61) et de 68% chez les chiennes non vaccinées (groupe placebo) (19/28) (test exact de Fisher, p=0,11).

Il doit être bien compris que l'invention définie par les revendications annexées n'est pas limitée aux modes de réalisation particuliers indiqués dans la description ci-dessus.

## Revendications

1. Utilisation d'antigène de l'herpès virus canin (CHV) pour la préparation d'un vaccin permettant de protéger les chiots contre l'herpèsvirose canine, destiné à être administré chez la chienne gestante environ 10 jours après la date de la saillie et environ 10 jours avant la date présumée de mise bas,
dans laquelle l'antigène comprend du virus CHV inactivé, ou une ou plusieurs sous-unités du virus CHV,
et dans laquelle le vaccin comprend une émulsion huile-dans-l'eau comprenant de l'huile de paraffine liquide légère.

2. Utilisation selon la revendication 1 dans laquelle les vaccins sont identiques.

3. Utilisation selon la revendication 1, dans laquelle les vaccins sont différents.

4. Utilisation selon l'une quelconque des revendications 1 à 3, dans laquelle le vaccin est un vaccin de sous-unités comprenant une glycoprotéine d'enveloppe du virus CHV.

5. Utilisation selon l'une quelconque des revendications 1 à 3, dans laquelle le vaccin comprend du virus CHV inactivé par un traitement chimique et/ou thermique.

6. Utilisation selon l'une quelconque des revendications 4 à 5, dans laquelle le vaccin comprend une quantité d'antigène correspondant à un équivalent en titre avant inactivation de 10⁵ à 10^{9,5} doses infectantes 50% en culture de cellules (DICC₅₀) par dose.

7. Utilisation selon la revendication 1 à 6, dans laquelle l'émulsion huile-dans-l'eau comprend des esters d'acides ou d'alcools à groupement alkyle linéaire.

8. Utilisation selon la revendication 7, dans laquelle les esters sont des huiles végétales, l'oléate d'éthyle, le di(caprylate/caprate) de propylène glycol, le tri (caprylate/caprate) de glycérol ou le dioléate de propylène glycol.

9. Utilisation selon la revendication 1 à 8, dans laquelle l'émulsion huile-dans-l'eau comprend des esters d'acides ou d'alcools gras ramifiés.

10. Utilisation selon la revendication 9, dans laquelle les esters sont des esters de l'acide isostéarique.

11. Utilisation selon l'une quelconque des revendications 1 à 9, dans laquelle le vaccin est formulé sous forme d'une émulsion comprenant de l'oléate d'anhydromannitol, de l'acide oléique éthoxylé et de l'huile de paraffine liquide légère.

12. Vaccin anti-CHV pour son utilisation dans la protection des chiots contre l'herpèsvirose canine, destiné à être administré chez la chienne gestante environ 10 jours après la date de la saillie et environ 10 jours avant la date présumée de mise bas, ledit vaccin comprenant les sous-unités comprenant une glycoprotéine d'enveloppe du virus CHV dans un véhicule ou excipient acceptable au plan vétérinaire, et un adjuvant acceptable sur le plan vétérinaire comprenant une huile de paraffine liquide légère.

13. Vaccin selon la revendication 12, dans lequel le vaccin est obtenu après élimination des capsides virales.

14. Vaccin selon la revendication 12 ou 13, dans lequel le vaccin est obtenu à partir d'un virus CHV inactivé par un traitement chimique et/ou thermique.

15. Vaccin selon la revendication 14, dans lequel le virus CHV est inactivé par le formol, la β-propiolactone, l'éthylèneimine, ou l'éthylèneimine binaire.

16. Vaccin selon l'une quelconque des revendications 12 à 15, dans lequel le vaccin est obtenu en soumettant une suspension de particules virales CHV à l'action d'un détergent approprié.

17. Vaccin selon l'une quelconque des revendications 12 à 16, comprenant des sous-unités du virus CHV en quantité qui équivaut à un équivalent titre avant inactivation de 10⁶ à 10^{9,5} DICC₅₀ par dose.

18. Vaccin selon la revendication 17, comprenant des sous-unités du virus CHV en quantité qui équivaut à un équivalent titre avant inactivation de 10⁷ à 10⁹ DICC₅₀ par dose.

19. Vaccin selon la revendication 18, comprenant des sous-unités du virus CHV en quantité qui équivaut à un équivalent titre avant inactivation de 10^{7,5} à 10^{8,5} par dose.

20. Vaccin selon l'une des revendications 12 à 19, comprenant un titre en glycoprotéine gB, mesuré en ELISA, de 0,01 µg à 30 µg, par dose.

21. Vaccin selon la revendication 20, comprenant un titre en glycoprotéine gB, mesuré en ELISA de 0,1 µg à 10 µg par dose.

22. Vaccin selon la revendication 21 comprenant un titre en glycoprotéine gB, mesuré en ELISA de 0,3 µg à 3 µg par dose.

23. Vaccin anti-CHV pour son utilisation dans la protection des chiots contre l'herpèsvirose canine, destiné à être administré chez la chienne gestante environ 10 jours après la date de la saillie et environ 10 jours avant la date présumée de mise bas, ledit vaccin comprenant du virus inactivé et une émulsion huile-dans-l'eau comprenant de l'huile de paraffine liquide légère.

24. Vaccin selon la revendication 23 dans lequel le virus est inactivé par un traitement chimique et/ou thermique.

25. Vaccin selon la revendication 24, dans lequel le virus CHV est inactivé par le formol, la β-propiolactone, l'éthylèneimine, ou l'éthylèneimine binaire.

26. Vaccin selon l'une quelconque des revendications 23 à 25, comprenant une quantité d'antigène qui équivaut à un équivalent titre avant inactivation de 10⁵ à 10⁹ DICC₅₀ par dose.

27. Vaccin selon la revendication 26, comprenant une quantité d'antigène qui équivaut à un équivalent titre avant inactivation de 10⁶ à 10⁸ DICC₅₀ par dose.

28. Vaccin selon l'une quelconque des revendications 23 à 27, dans lequel l'émulsion huile-dans-l'eau comprend, des esters d'acides ou d'alcools à groupement alkyle linéaire.

29. Vaccin selon la revendication 28, dans lequel les esters sont des huiles végétales, l'oléate d'éthyle, le di(caprylate/caprate) de propylène glycol, le tri(caprylate/caprate) de glycérol ou le dioléate de propylène glycol.

30. Vaccin selon l'une quelconque des revendications 23 à 29, dans lequel l'émulsion huile-dans-l'eau comprend des esters d'acides ou d'alcools gras ramifiés.

31. Vaccin selon la revendication 30, dans lequel les esters sont des esters de l'acide isostéarique.

32. Vaccin selon l'une quelconque des revendications 23 à 31, dans lequel l'émulsion huile dans l'eau comprend de l'oléate d'anhydromannitol, de l'acide oléique éthoxylé et de l'huile de paraffine liquide légère.

## Claims

1. Use of a Canine Herpes Virus (CHV) antigen for the preparation of a vaccine for protecting puppies against Canine HerpesVirosis, which is intended to be administered to the gestating bitch about 10 days after insemination and about 10 days before the expected date of whelping,
wherein the CHV antigen comprises an inactivated CHV or one or several CHV subunits,
and wherein the vaccine comprises an oil-in-water based emulsion comprising light liquid paraffin oil.

2. Use according to claim 1, wherein both vaccines are identical.

3. Use according to claim 1, wherein both vaccines are different.

4. Use according to any one of claims 1 to 3, wherein the vaccine is a subunit vaccine comprising an envelope glycoprotein of CHV.

5. Use according to any one of claims 1 to 3, wherein the vaccine comprises CHV which has been inactivated by a chemical and/or heat treatment.

6. Use according to any one of claims 4 or 5, wherein the vaccine comprises an amount of antigen equivalent to a titre before inactivation of 10⁵ to 10^{9.5} Cell Culture 50% Infective Doses (CCID₅₀) per dose.

7. Use according to any one of claims 1 to 6, wherein the oil-in-water emulsion comprises esters of acids or of alcohols containing a linear alkyl group.

8. Use according to claim 7, wherein the esters are plant oils, ethyl oleate, propylene glycol di(caprylate/caprate), glycerol tri (caprylate/caprate) or propylene glycol dioleate.

9. Use according to any one of claims 1 to 8, wherein the oil-in-water emulsion comprises esters of branched fatty acids or alcohols.

10. Use according to claim 9, wherein the esters are isostearic acid esters.

11. Use according to any one of claims 1 to 9, wherein the vaccine is formulated in the form of an emulsion comprising anhydromannitol oleate, ethoxylated oleic acid and light liquid paraffin oil.

12. Anti-CHV vaccine for use in the protection of puppies against Canine HerpesVirosis, which is intended to be administered to the gestating bitch about 10 days after insemination and about 10 days prior to whelping, comprising the subunits comprising an envelope glycoprotein of CHV in a veterinarilly acceptable vehicle or excipient and a veterinarilly acceptable adjuvant comprising light liquid paraffin oil.

13. Vaccine according to claim 12, wherein the vaccine is obtained after elimination of the viral capsids.

14. Vaccine according to claim 12 or 13, wherein the vaccine is obtained from CHV which has been inactivated by a chemical and/or heat treatment.

15. Vaccine according to claim 14, wherein CHV is inactivated by formalin, β-propiolactone, ethyleneimine, or binary ethyleneimine.

16. Vaccine according to any one of claims 12 to 15, wherein the vaccine is obtained by subjecting a suspension of CHV viral particles to the action of a suitable detergent.

17. Vaccine according to any one of claims 12 to 16, comprising subunits of CHV in an amount equivalent to a titre before inactivation of 10⁶ to 10^{9.5} CCID₅₀ per dose.

18. Vaccine according to claim 17, comprising subunits of CHV in an amount equivalent to a titre before inactivation of 10⁷ to 10⁹ CCID₅₀ per dose.

19. Vaccine according to claim 18, comprising subunits of CHV in an amount equivalent to a titre before inactivation of 10^{7.5} to 10^{8.5} per dose.

20. Vaccine according to any one of claims 12 to 19, comprising a glycoprotein gB titre, as measured by ELISA, of 0.01 µg to 30 µg per dose.

21. Vaccine according to claim 20, comprising a glycoprotein gB titre, as measured by ELISA, of 0.1 µg to 10 µg per dose.

22. Vaccine according to claim 21, comprising a glycoprotein gB titre, as measured by ELISA, of 0.3 µg to 3 µg per dose.

23. Anti-CHV vaccine for use in the protection of puppies against Canine HerpesVirosis, which is intended to be administered to the gestating bitch about 10 days after insemination and about 10 days prior to whelping, comprising inactivated CHV and an oil-in-water emulsion comprising light liquid paraffin oil.

24. Vaccine according to claim 23, wherein the virus is inactivated by a chemical and/or heat treatment.

25. Vaccine according to claim 24, wherein the CHV virus is inactivated by formalin, β-propiolactone, ethyleneimine, or binary ethyleneimine.

26. Vaccine according to any one of claims 23 to 25, comprising an amount of antigen equivalent to a titre before inactivation of 10⁵ to 10⁹ CCID₅₀ per dose.

27. Vaccine according to claim 26, comprising an amount of antigen equivalent to a titre before inactivation of 10⁶ to 10⁸ CCID₅₀ per dose.

28. Vaccine according to any one of claims 23 to 27, wherein the oil-in-water emulsion comprises esters of acids or of alcohols containing a linear alkyl group.

29. Vaccine according to claim 28, wherein the esters are plant oils, ethyl oleate, propylene glycol di(caprylate/caprate), glycerol tri (caprylate/caprate) or propylene glycol dioleate.

30. Vaccine according to any one of claims 23 to 29, wherein the oil-in-water emulsion comprises esters of branched fatty acids or alcohols.

31. Vaccine according to claim 30, wherein the esters are isostearic acid esters.

32. Vaccine according to any one of claims 23 to 31, wherein the oil-in-water emulsion comprises anhydromannitol oleate, ethoxylated oleic acid and light liquid paraffin oil.

## Patentansprüche

1. Verwendung eines Antigens des Hundeherpesvirus (CHV) zur Herstellung eines Impfstoffs, mit dem Welpen vor Hundeherpes-Virose geschützt werden können, für die Verabreichung an die tragende Hündin etwa 10 Tage nach dem Deckdatum und etwa 10 Tage vor dem vermutlichen Wurfdatum,
- wobei das Antigen inaktiviertes CHV-Virus oder eine oder mehrere Untereinheiten des CHV-Virus umfasst
- und wobei der Impfstoff eine Öl-in-Wasser-Emulsion umfasst, die dünnflüssiges Paraffin umfasst.

2. Verwendung nach Anspruch 1, wobei die Impfstoffe identisch sind.

3. Verwendung nach Anspruch 1, wobei die Impfstoffe unterschiedlich sind.

4. Verwendung nach einem der Ansprüche 1 bis 3, wobei es sich bei dem Impfstoff um einen Impfstoff aus Untereinheiten handelt, der ein Hüllglykoprotein des CHV-Virus umfasst.

5. Verwendung nach einem der Ansprüche 1 bis 3, wobei der Impfstoff das CHV-Virus umfasst, das durch eine chemische und/oder thermische Behandlung inaktiviert wurde.

6. Verwendung nach einem der Ansprüche 4 bis 5, wobei der Impfstoff eine Menge an Antigen umfasst, die einem Äquivalent in Bezug auf den Titer vor der Inaktivierung von 10⁵ bis 10⁹,⁵ 50%-Zellkultur-Infektionsdosen (CCID50) pro Dosis entspricht.

7. Verwendung nach Anspruch 1 bis 6, wobei die Öl-in-Wasser-Emulsion Ester von Säuren oder Alkoholen mit einer geraden Alkylgruppe umfasst.

8. Verwendung nach Anspruch 7, wobei es sich bei den Estern um pflanzliche Öle, Ethyloleat, Propylenglykoldi(caprylat/caprat), Glycerintri((caprylat/caprat) oder Propylenglykoldioleat handelt.

9. Verwendung nach Anspruch 1 bis 8, wobei die Öl-in-Wasser-Emulsion Ester von verzweigten Fettsäuren oder Fettalkoholen umfasst.

10. Verwendung nach Anspruch 9, wobei es sich bei den Estern um Isostearinsäureester handelt.

11. Verwendung nach einem der Ansprüche 1 bis 9, wobei der Impfstoff in Form einer Emulsion formuliert wird, die Anhydromannitoleat, ethoxylierte Oleinsäure und dünnflüssiges Paraffin umfasst.

12. Anti-CHV-Impfstoff für die Verwendung zum Schutz von Welpen vor Hundeherpes-Virose zur Verabreichung an die tragende Hündin etwa 10 Tage nach dem Deckdatum und etwa 10 Tage vor dem vermutlichen Wurfdatum, wobei der Impfstoff die Untereinheiten, die ein Hüllglykoprotein des CHV-Virus umfassen, in einem veterinärmedizinisch annehmbaren Vehikel oder Excipienten und einen veterinärmedizinisch annehmbaren Hilfsstoff umfasst, der ein dünnflüssiges Paraffin umfasst.

13. Impfstoff nach Anspruch 12, wobei der Impfstoff nach Beseitigung der Virus-Capside erhalten wird.

14. Impfstoff nach Anspruch 12 oder 13, wobei der Impfstoff ausgehend von einem durch chemische und/oder thermische Behandlung inaktivierten CHV-Virus erhalten wird.

15. Impfstoff nach Anspruch 14, wobei das CHV-Virus durch Formol, β-Propiolacton, Ethylenimin oder binäres Ethylenimin inaktiviert wird.

16. Impfstoff nach einem der Ansprüche 12 bis 15, wobei der Impfstoff erhalten wird, indem man eine Suspension von CHV-Viruspartikeln der Einwirkung eines geeigneten Detergenzes unterwirft.

17. Impfstoff nach einem der Ansprüche 12 bis 16, der die Untereinheiten des CHV-Virus in einer Menge umfasst, die einem Äquivalent in Bezug auf den Titer vor der Inaktivierung von 10⁶ bis 10^{9,5} CCID50 pro Dosis entspricht.

18. Impfstoff nach Anspruch 17, der die Untereinheiten des CHV-Virus in einer Menge umfasst, die einem Äquivalent in Bezug auf den Titer vor der Inaktivierung von 10⁷ bis 10⁹ CCID50 pro Dosis entspricht.

19. Impfstoff nach Anspruch 18, der die Untereinheiten des CHV-Virus in einer Menge umfasst, die einem Äquivalent in Bezug auf den Titer vor der Inaktivierung von 10^{7,5} bis 10^{8,5} CCID50 pro Dosis entspricht.

20. Impfstoff nach einem der Ansprüche 12 bis 19, der einen mittels ELISA gemessenen Titer an Glykoprotein gB von 0,01 µg bis 30 µg pro Dosis umfasst.

21. Impfstoff nach Anspruch 20, der einen mittels ELISA gemessenen Titer an Glykoprotein gB von 0,1 µg bis 10 µg pro Dosis umfasst.

22. Impfstoff nach Anspruch 21, der einen mittels ELISA gemessenen Titer an Glykoprotein gB von 0,3 µg bis 3 µg pro Dosis umfasst.

23. Anti-CHV-Impfstoff für die Verwendung zum Schutz von Welpen vor Hundeherpes-Virose zur Verabreichung an die tragende Hündin etwa 10 Tage nach dem Deckdatum und etwa 10 Tage vor dem vermutlichen Wurfdatum, wobei der Impfstoff inaktiviertes Virus und eine Öl-in-Wasser-Emulsion umfasst, die dünnflüssiges Paraffin umfasst.

24. Impfstoff nach Anspruch 23, wobei das Virus durch chemische und/oder thermische Behandlung inaktiviert wird.

25. Impfstoff nach Anspruch 24, wobei das CHV-Virus durch Formol, β-Propiolacton, Ethylenimin oder binäres Ethylenimin inaktiviert wird.

26. Impfstoff nach einem der Ansprüche 23 bis 25, der eine Menge an Antigen umfasst, die einem Äquivalent in Bezug auf den Titer vor der Inaktivierung von 10⁵ bis 10⁹ CCID50 pro Dosis entspricht.

27. Impfstoff nach Anspruch 26, der eine Menge an Antigen umfasst, die einem Äquivalent in Bezug auf den Titer vor der Inaktivierung von 10⁶ bis 10⁸ CCID50 pro Dosis entspricht.

28. Impfstoff nach einem der Ansprüche 23 bis 27, wobei die Öl-in-Wasser-Emulsion Ester von Säuren oder Alkoholen mit einer geraden Alkylgruppe umfasst.

29. Impfstoff nach Anspruch 28, wobei es sich bei den Estern um pflanzliche Öle, Ethyloleat, Propylenglykoldi(caprylat/caprat), Glycerintri((caprylat/caprat) oder Propylenglykoldioleat handelt.

30. Impfstoff nach einem der Ansprüche 23 bis 29, wobei die Öl-in-Wasser-Emulsion Ester von verzweigten Fettsäuren oder Fettalkoholen umfasst.

31. Impfstoff nach Anspruch 30, wobei es sich bei den Estern um Isostearinsäureester handelt.

32. Impfstoff nach einem der Ansprüche 23 bis 31, wobei die Öl-in-Wasser-Emulsion Anhydromannitoleat, ethoxylierte Oleinsäure und dünnflüssiges Paraffin umfasst.
